# EUROPEAN PATENT SPECIFICATION

(11) **EP 3 074 090 B1**
(45) Date of publication and mention of the grant of the patent: **22.11.2017**
(21) Application number: 15703861.3
(22) Date of filing: 30.01.2015
(51) Int. Cl.: A61N 7/02, A61N 7/00, A61B 18/00, A61B 34/20, A61B 90/00

(54) **DEVICE FOR ABLATION AND PHOTOACOUSTICS IMAGING**
VORRICHTUNG ZUR ABLATION UND FOTOAKUSTISCHEN BILDGEBUNG
DISPOSITIF POUR ABLATION ET IMAGERIE PHOTO-ACOUSTIQUE

(30) Priority: 10.02.2014 US 201461937911 P
(43) Date of publication of application: 05.10.2016
(73) Proprietor: St. Jude Medical, Cardiology Division, Inc., St. Paul, MN 55117 (US)
(72) Inventor: LUPOTTI, Fermin A., Lake Forest, California 92630 (US)
(74) Representative: Kramer Barske Schmidtchen Patentanwälte PartG mbB
(86) International application number: PCT/US2015/013834
(87) International publication number: WO 2015/119861

(56) References cited:
- WO-A1-2013/018003
- WO-A1-2013/123014
- WO-A2-2012/120495
- US-B2- 8 617 148

## Description

### BACKGROUND OF THE DISCLOSURE

### a. Field of the Disclosure

This disclosure relates generally to devices, systems and methods for providing ablation therapy to tissue, assessing the effects of such ablation therapy by measuring a photoacoustic response from the tissue, and/or imaging tissue and other anatomical structures within the body. In particular, the instant disclosure relates to an ultrasound transducer, adapted for use with such devices, systems and methods, that can be used for both ablation therapy as well as imaging and assessing various characteristics of tissue during such ablation therapy.

### b. Background Art

Ablation therapy can be used to treat cardiac arrhythmias (including, but not limited to, atrial fibrillation, atrial flutter, atrial tachycardia, and ventricular tachycardia), hypertension, and various forms of cancer. An ablation catheter delivers ablative energy (e.g., radiofrequency energy, light energy, ultrasound, microwave, or thermal (cryo or heat based) energy) to tissue to create a lesion in the tissue. In the case of cardiac tissue, the lesions disrupt undesirable electrical pathways that lead to arrhythmias. As a treatment for hypertension, ablation can be used to create lesions in renal nerves, resulting in renal denervation and reduced blood pressure. Similarly, ablation can be used to create lesions in tumors, such as those occurring in the prostate.

To be effective, ablation therapy must be provided in the right amount and at the right location. Delivering too little ablative energy to tissue results in ineffective therapy; and delivering too much ablative energy can cause serious complications, such as perforation of tissue or formation of a blood thrombus. Furthermore, failure to deliver ablative energy at the correct location may result in ineffective therapy and may unnecessarily damage tissue.

Because of the negative consequences of delivering ablation therapy in the wrong amount or at the wrong location, it is desirable to continuously monitor the effects of ablation energy on the tissue to assess the effectiveness of the therapy. Current monitoring techniques such as measuring tissue elasticity, echogenicity, and speed of sound, however, have limited benefits because these techniques pose difficulty in identifying lesion boundaries and often rely on sensing mechanisms located far from the site of the ablation.

WO 2012/120495 A2 relates to treatment of tissue and monitoring application of energy.

WO 2013/123014 A1 relates to a catheter, system and method for assessing the effect of ablation therapy in which an electromagnetic radiation emitter such as an optic fiber is used to deliver electromagnetic radiation to tissue, without an ultrasound transducer operable in an imaging mode and an ablation mode.

US 8,617,148 B2 relates to tissue ablation device of any type for forming lesion in tissue without a tracking module for identifying and tracking a region of interest and confirming that the tissue remains within the region of interest.

WO 2013/018003 A1 relates to a method, system and program product for accurately visualizing soft tissue motion on an X-ray image by defining a point of interest and determining motion of the selected point from real time ultrasound images.

### BRIEF SUMMARY OF THE DISCLOSURE

It is desirable to assess the effects of ablation therapy using an ultrasound transducer that can be used for imaging, tissue assessment, and ablation therapy.

The invention is defined by the appended independent claim 1, preferred embodiments are described in the dependent claims.

A method for assessing effects of ablation therapy on tissue in a body in accordance with one embodiment of the present teachings includes acquiring at least one ultrasound image of a region of interest, tracking the region of interest, delivering ultrasound ablative energy from an ultrasound transducer to the region of interest, emitting electromagnetic radiation from an optic fiber to cause generation of a photoacoustic wave from tissue in the region of interest, receiving a tissue response signal from the ultrasound transducer indicative of a characteristic of tissue responsive to the electromagnetic radiation, and analyzing the tissue response signal to determine at least one characteristic of the tissue responsive to the ablation therapy.

A system for assessing effects of ablation therapy on tissue in a body in accordance with another embodiment of the present teachings includes a catheter comprising an elongate shaft having a proximal section and a distal section; and an ultrasound transducer disposed at the distal section of the shaft, the ultrasound transducer configured to deliver ultrasound ablative energy and to transmit and receive ultrasound waves that can be used to generate ultrasound images. The system further includes an optic fiber disposed within the shaft of the first catheter or another catheter and configured to emit electromagnetic radiation at the distal end of the shaft. The system further includes an electronic control unit (ECU) in communication with the first catheter. The ECU includes an imaging module configured to cause the ultrasound transducer to generate and acquire at least one ultrasound image from a region of interest within the body; a tracking module configured to identify and track the region of interest using at least one ultrasound image acquired with the ultrasound transducer; a therapy module configured to generate a signal to cause the ultrasound transducer to deliver ultrasound ablative energy to the region of interest; a radiation module configured to generate a signal to cause the optic fiber to emit electromagnetic radiation to cause generation of a photoacoustic wave from tissue in the region of interest; a tissue sensing module configured to receive a tissue response signal indicative of a characteristic of tissue responsive to the electromagnetic radiation; and an analysis module configured to analyze the tissue response signal to determine at least one characteristic of the tissue responsive to the ablation therapy.

A system for assessing the effects of ablation therapy on tissue in a body in accordance with another embodiment of the present teachings includes an ultrasound transducer configured to generate ultrasound images and to deliver ultrasound ablative energy to tissue. The system further includes an optic fiber configured to emit electromagnetic radiation to the tissue. The system further includes an ECU including the following: an imaging module configured to operate the ultrasound transducer to generate and acquire at least one ultrasound image from a region of interest within the body; a tracking module configured to identify and track the region of interest using at least one ultrasound image acquired by the ultrasound transducer; a therapy module configured to generate a signal to cause the ultrasound transducer to deliver ultrasound ablative energy to the region of interest; a radiation module configured to generate a signal to cause the optic fiber to emit electromagnetic radiation to cause generation of a photoacoustic wave from tissue in the region of interest; a tissue sensing module configured to receive a tissue response signal indicative of a characteristic of tissue responsive to the electromagnetic radiation; and an analysis module configured to analyze the tissue response signal to determine at least one characteristic of the tissue responsive to the ablation therapy.

### BRIEF DESCRIPTION OF THE DRAWINGS

Figure 1 is a diagrammatic view of an exemplary system for delivery of ablation therapy to tissue in a body and for assessing the effects of the ablation therapy in accordance with one embodiment of the present teachings.
Figure 2 is a diagrammatic view of an exemplary catheter for use with the system of Figure 1 according to one embodiment.
Figure 3 is a diagrammatic view of an exemplary catheter for use with the system of Figure 1 according to another embodiment.
Figure 4 is a diagrammatic view showing the catheter of Figure 2 implanted within a blood vessel.
Figure 5 is a diagrammatic view of the electronic control unit of the system of Figure 1.
Figure 6 is a diagrammatic view showing the transducer of the catheter of Figure 2 implanted within the heart.
Figure 7 is a flow chart diagram illustrating a method for delivery of ablation therapy to tissue in a body and for assessing the effects of the ablation therapy in accordance with one embodiment of the present teachings.

### DETAILED DESCRIPTION OF THE DISCLOSURE

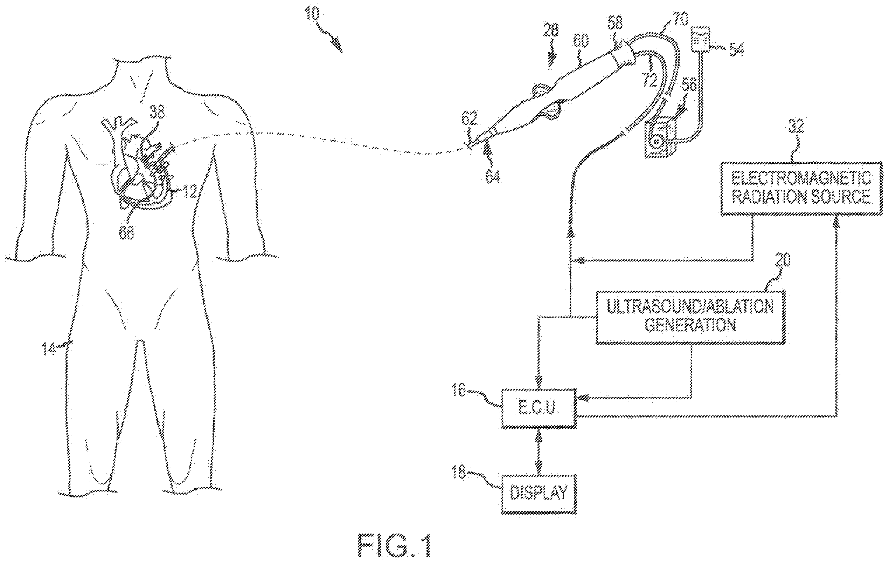

Referring now to the drawings, wherein like reference numerals are used to identify identical components in the various views, Figure 1 illustrates a system 10 for delivery of ablation therapy to tissue 12 in a body 14 and for imaging tissue and assessing the effects of the ablation therapy in accordance with one embodiment of the present teachings, and as described in commonly assigned Published PCT Patent Application No. WO 2013/123014 A1. Although the illustrated system 10 relates to diagnosis and treatment of cardiac tissues, it should be understood that the present invention may find application in the diagnosis and treatment of a variety of tissues, such as renal or prostate tissue, for example. System 10 may include an electronic control unit (ECU) 16, a display 18, an ultrasound/ablation generator 20, a catheter 28, and an electromagnetic radiation source 32 that generates electromagnetic radiation that may comprise, for example, visible, near infrared (NIR), or short wave infrared (SWIR) light.

ECU 16 is provided to control the operation of ultrasound/ablation generator 20 and electromagnetic radiation source 32, and to process signals generated by an ultrasound transducer 38, positioned at the distal end 66 of the shaft 62 of catheter 28, to assess delivery of ablation therapy to tissue 12. Ultrasound transducer 38, when operating as an intracardiac echocardiography (ICE) transducer, can also be used to acoustically image a region of interest. ECU 16 may be specific to system 10 or may be used in the control of other conventional systems in an electrophysiology (EP) lab including, for example, medical device position, navigation and/or visualization systems, imaging systems, EP monitoring systems and other systems. ECU 16 may comprise a programmable microprocessor or microcontroller or may comprise an application specific integrated circuit (ASIC). ECU 16 may include a central processing unit (CPU) and an input/output (I/O) interface through which ECU 16 may receive a plurality of input signals, including signals from transducer 38, and generate a plurality of output signals including those used to control display 18, generator 20, and radiation source 32.

Display 18 is provided to convey real time imaging and other information to a physician to assist in diagnosis and treatment of tissue 12. Display 18 may comprise a conventional computer monitor or other display device. Display 18 may comprise a portion of the intracardiac ultrasound console sold under the trademark "VIEWMATE Z" by St. Jude Medical, Inc. of St. Paul, Minnesota. Display 18 may provide a variety of information to the physician including images of tissue 12, the response of tissue 12 to ablation therapy, and lesion assessment information. Display 18 may also present a graphical user interface (GUI) to the physician.

Ultrasound/ablation generator 20 is provided to control generation of ultrasound and may operate under the control of ECU 16. Generator 20 can deliver ultrasound energy for imaging purposes, and it can also deliver high intensity focused ultrasound (HIFU) energy for ablative purposes. An operator and/or ECU 16 can cause generator 20 to alternate between delivery of ultrasound energy in an imaging mode and delivery of HIFU in an ablation mode. Further, the operator and/or ECU 16 can control the duration and frequency of the imaging and ablation modes of generator 20.

Ultrasound transducer 38 is provided to convert electrical signals into ultrasound signals transmitted to tissue 12 and to convert reflected ultrasound signals from tissue 12 into electrical signals for processing by ECU 16 and imaging of tissue 12. Transducer 38 is conventional in the art and is disposed at distal end 66 of shaft 62.

Catheter 28 may be connected to a fluid source 54 having a biocompatible fluid such as saline through a pump 56 (which may comprise, for example, a fixed rate roller or peristaltic pump or variable volume syringe pump with a gravity feed supply from fluid source 54 as shown) for irrigation and to cool down the ultrasound transducer. Catheter 28 may include a cable connector or interface 58, a handle 60, a shaft 62 having a proximal end 64 and a distal end 66 and one or more diagnostic or treatment elements supported thereon. Connector 58 may provide mechanical, fluid and electrical connection(s) for fluid conduit 70 extending from pump 56, and a cable 72 extending from ultrasound/ablation generator 20. Connector 58 is conventional in the art and is disposed at a proximal end of catheter 28. Catheter 28 may also include other conventional components not illustrated herein such as a temperature sensor, additional electrodes, one or more position sensors, and corresponding conductors or leads.

Handle 60 provides a location for the physician to hold catheter 28 and may further provide means for steering or guiding shaft 62 within body 14. For example, handle 60 may include means to actuate various steering wires (not shown) extending to distal end 66 of shaft 62 to control translation and/or deflection of shaft 62. Handle 60 may also be conventional in the art, and it will be understood that the construction of handle 60 may vary. It should be understood that catheter 28 may be manipulated manually by a physician using handle 60 or automatically through, for example, robotic controls.

Shaft 62 provides structural support to the other components of catheter 28 and may also permit transport, delivery and/or removal of fluids (including irrigation fluids and bodily fluids), medicines, and/or surgical tools or instruments to and from tissue 12. Shaft 62 is an elongate, tubular, flexible/deformable member configured for movement within body 14. Shaft 62 may be introduced into a blood vessel or other structure within body 14 through a conventional introducer. Shaft 62 may then be steered or guided through body 14 to a desired location such as tissue 12 with a guiding introducer such as the Agilis™ NxT steerable introducer available from St. Jude Medical, Inc. or with guide wires or other means known in the art. Shaft 62 may be made from conventional polymeric materials such as polyurethane, polyfluoroethylene (PTFE) including PTFE sold under the registered trademark "TEFLON" by E.I. DuPont de Nemours & Co. Corp., polyether block amides, and nylon or thermoplastic elastomers such as the elastomer sold under the registered trademark "PEBAX" by Arkema, Inc. In addition to supporting steering wires (not shown), shaft 62 supports associated conductors, and possibly additional electronics used for signal processing or conditioning. Shaft 62 further defines one or more lumens configured to house conductors and provide irrigation fluid from fluid source 54 to an external surface of ablation delivery.

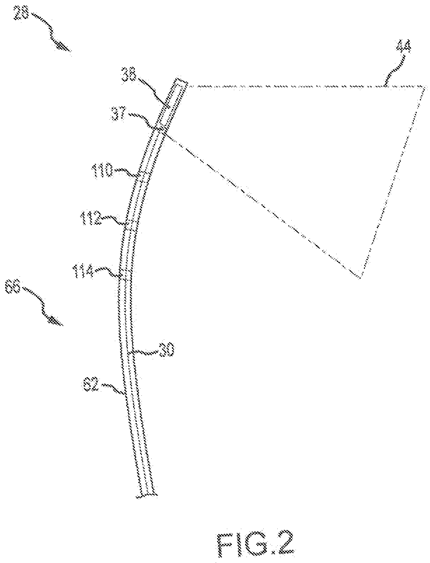

Figure 2 is a diagrammatic view of the shaft 62 of an exemplary catheter 28 (see Figure 1) for use with the system 10 of Figure 1. In one embodiment, shaft 62 supports an optic fiber 30, which can emit electromagnetic radiation to cause generation of a photoacoustic wave from tissue. The optic fiber 30 can be configured to run through shaft 62 from the proximal end 64 (see Figure 1) to light radiation port 37 near the ultrasound transducer 38, such that there is a clear path for light to illuminate the surrounding tissue in the direction of the ultrasound imaging plane (or in any other direction). In other embodiments, optic fiber 30 can be disposed within the shaft of a second catheter (not shown), separate from catheter 28.

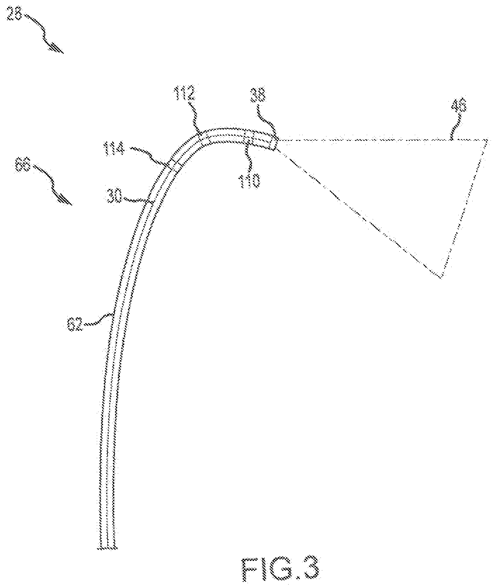

As can be seen in Figure 2, the shaft 62 includes a proximal section (not shown) and a distal section 66. An ultrasound transducer 38 located on the distal section 66 of the shaft 62 of catheter 28 is configured to transmit and receive ultrasound signals in a lateral direction (or a direction that is generally perpendicular to a longitudinal axis of shaft 62) creating a field of view or imaging plane 44 that is generally perpendicular to the longitudinal axis of shaft 62. Referring to Figure 3, in another embodiment, transducer 38 may transmit and receive ultrasound signals in a direction that is generally parallel to a longitudinal axis of shaft 62 creating a field of view or imaging plane 46 that is generally parallel to a longitudinal axis of shaft 62.

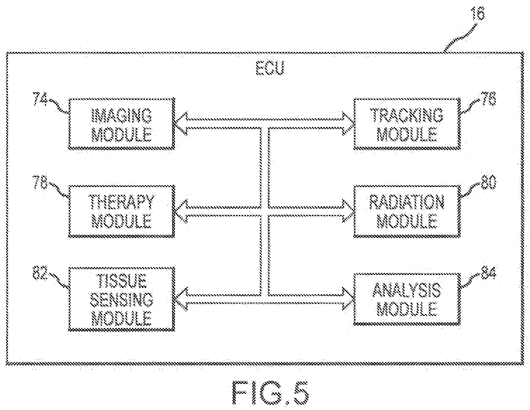

In an embodiment, position or orientation sensors can be located on the distal section 66 of shaft 62, proximal to transducer 38. Examples of such sensors include position-sensing ring electrodes 110, 112, and 114, shown in Figures 2 and 3. Electrodes 110, 112, and 114 can be used to detect the position of the distal section 66 of shaft 62, such as described, for example, in commonly owned U.S. Patent No. 7,263,397. For example, electrodes 110, 112, and 114 can be used to determine whether the ultrasound transducer 38 transmits and receives ultrasound signals in a direction that is parallel or perpendicular to a longitudinal axis of shaft 62. In addition, electrodes 110, 112, and 114 can be used to detect and track a region of interest for a therapy, such as ablation, as described further with respect to Figure 5 below.

In accordance with one aspect of the present teachings, transducer 38 is configured to deliver ultrasound ablative energy, such as HIFU, for example. In accordance with another aspect of the present teachings, transducer 38 is configured to receive one or more photoacoustic waves, as described in greater detail below. Transducer 38 may alternate between the transmission and receipt of ultrasound signals for regular pulse-echo imaging, the delivery of ablative energy, and the capture of photoacoustic signals for tissue evaluation at predetermined time periods. Such time periods can be controlled by an operator or by ECU 16, for example.

It should be noted that transducer 38 (and the distal end 66 of shaft 62) may be disposed at some distance from the site of ablation, as ablation assessment is performed via an analysis of the photoacoustic response of tissue to electromagnetic radiation. For example, transducer 38 may be disposed in any chamber of the heart (including right atrium, left atrium, or a ventricle) and locations exterior to the heart before assessing ablation taking place in any chamber of the heart (including right atrium, left atrium, or a ventricle). Transducer 38 may also be disposed in body cavities exterior to the heart, such as veins, arteries, or gastrointestinal ducts, in order to view the kidney or prostate gland, for example. In one embodiment, for example, the catheter 28 is configured for use in the renal arteries for assessing one or more characteristics of the arterial tissue during a renal denervation procedure.

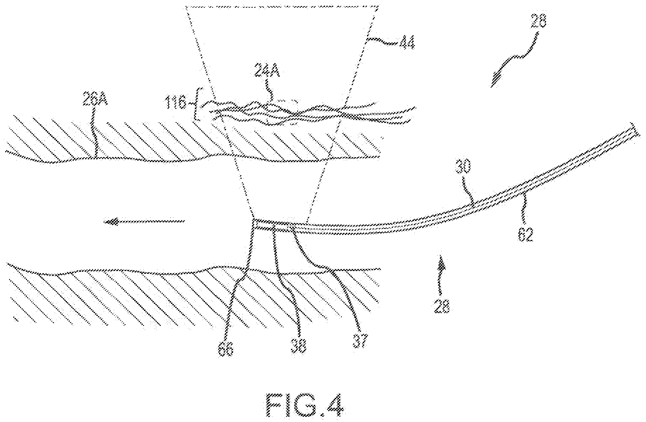

Figure 4 is a diagrammatic view showing the exemplary catheter 28 of Figure 2 with the transducer 38 (at the distal end 66 of shaft 62) disposed within a blood vessel 26A and targeting a region of interest (ROI) 24A within tissue 12 in imaging plane 44. In an embodiment, blood vessel 26A can be a renal artery and ROI 24A can include renal nerve bundles 116 in or near the vascular wall. Renal denervation, a procedure for treating resistant hypertension, can be carried out by providing HIFU ablative energy via transducer 38 to renal nerve bundles 116 via the renal artery. This can result in decreased renal sympathetic nerve activity and, therefore, decreased blood pressure. It should be noted, however, that blood vessel 26A can be another artery besides the renal artery, such as the aorta or a femoral artery, for example.

Figure 5 is a diagrammatic view illustrating an example of ECU 16 in greater detail. As can be seen in Figure 5, ECU 16 can include an imaging module 74, a tracking module 76, a therapy module 78, a radiation module 80, a tissue sensing module 82, and an analysis module 84, all of which can be in communication with each other. In some embodiments, ECU 16 can further be adapted to operate in three modes: an imaging mode, a therapy (or ablation) mode, and a lesion assessment mode. Imaging module 74 and tracking module 76 can be active during the imaging mode, therapy module 78 can be active during the therapy mode, and radiation module 80, tissue sensing module 82, and analysis module 84 can be active during the lesion assessment mode. Although imaging and ablation therapy can be performed simultaneously by transducer 38 in the present disclosure, multiple adjacent ultrasonic transducers can also be used, one operating in an imaging and/or assessment mode and another operating in a therapy mode, for example.

During the imaging mode, the imaging module 74 can be configured to cause ultrasound/ablation generator 20 to deliver ultrasound energy via transducer 38 to a ROI within the tissue 12 of body 14 to acquire at least one 2D (or 3D) ultrasound image. The tracking module 76 can cause ECU 16 to identify and track the ROI using 2D and/or 3D ultrasound images and software algorithms, for example, so as to ensure that the target tissue remains in the ROI. Tracking module 76 can also use position sensing electrodes 110, 112, and 114 (see Figures 2 and 3) to identify and track the ROI. In an embodiment, ROI tracking can be performed via image speckle or feature tracking between consecutive ultrasound images. If the ROI cannot be found within an image, the tracking module 76 can be configured to signal the transducer 38 (e.g. via the therapy module 78) to stop the delivery of HIFU ablative energy. The tracking module 76 can also be operator controlled.

During the therapy mode, the therapy module 78 can generate a signal to cause transducer 38 to deliver (or stop delivering) HIFU ablative energy to the ROI. The therapy module 78 can set the energy level for the HIFU ablation energy and determine an initial ablation period, for example. Such parameters can be adjustable and can be initially based on empirical data.

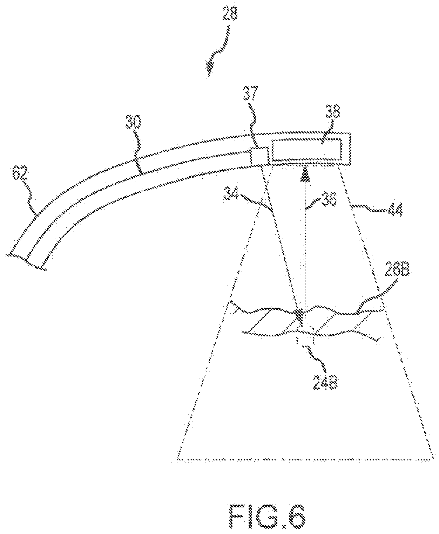

During the lesion assessment mode, the radiation module 80 can generate a signal to cause the optic fiber 30 to emit electromagnetic radiation to cause generation of a photoacoustic wave from the target tissue, which can then be received by transmitter 38. Figure 6 illustrates an exemplary embodiment in which electromagnetic radiation, shown as arrow 34 in this figure, is emitted from light radiation port 37, where optic fiber 30 terminates. The electromagnetic radiation 34 targets ROI 24B, which in this embodiment is located in a chamber of the heart (tissue 26B), such as an atrium or ventricle. The electromagnetic radiation 34 causes generation of a photoacoustic wave, shown here as arrow 36, from tissue in ROI 24B. The photoacoustic wave 36 is received by transducer 38 and communicated to tissue sensing module 82.

Referring back to Figure 5, the tissue sensing module 82 can generate a tissue response signal indicative of a characteristic of tissue responsive to the electromagnetic radiation. The tissue response signal can be one or more colors, for example, displayed on display 18 along with the 2D and/or 3D ultrasound images. The colors can vary in intensity and can be indicative of the magnitude, frequency, or location, for example, of the photoacoustic wave generated by the target tissue and received by transducer 38 (e.g., a light blue color could be used to represent a low frequency photoacoustic wave and a dark blue color could be used to represent a high frequency photoacoustic wave).

Finally, the analysis module 84 can be configured to analyze - via color intensity or shape, for example - the tissue response signal to determine at least one characteristic of the tissue responsive to the ablation therapy. In an example, analysis of the tissue response signal can include overlaying the tissue response signal and the 2D and/or 3D ultrasound image. An operator and/or ECU 16 can then use the resulting image to decide whether ablation is complete. The determined tissue characteristics - including lesion size (e.g., volume), depth, type and/or location, for example - can be displayed on display 18. A transmural lesion, for example, can be preferred for indicating that ablation is complete, as transmural lesions tend to prevent intima tissue damage.

In an embodiment, the therapy module 78 and the radiation module 80 are configured to operate during different time periods, such that lesion assessment does not occur until therapy is complete or temporarily stopped. An operator can determine these time periods, and ECU 16 can also be configured to control the time periods. In another embodiment, tissue sensing module 82 can be adapted to use the tissue response signal to control the therapy module 78. For example, if the tissue response signal suggests that a particular lesion needs to be made deeper in order to be transmural (e.g., based on input from the analysis module 84), the tissue sensing module 82 can signal the therapy module 78 to cause transducer 38 to deliver more HIFU ablative energy to the target tissue.

Although the above described system uses photoacoustic imaging for lesion assessment, it should be understood that other methodologies, such as measuring tissue elasticity, thermal strain or shear wave imaging, can be used for lesion assessment in accordance with the teachings of the present disclosure.

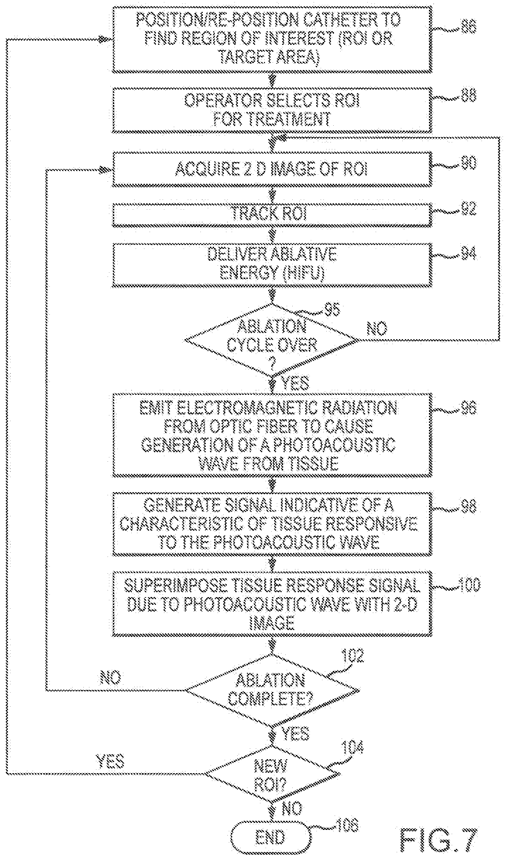

Figure 7 is a flow chart illustrating a method for assessing the effects of ablation therapy in accordance with one embodiment of the present teachings. The method can begin with step 86 of positioning the catheter to find a ROI where a target tissue is located and ablative treatment is to take place. At step 88, the operator and/or ECU 16 can select a ROI for treatment, such as an ROI at within a chamber of the heart or a blood vessel. At step 90, a 2D ultrasound image of the ROI can be obtained via delivery of ultrasound energy from generator 20, responsive to control by the operator and/or ECU 16. Next, at step 92, the ROI can be tracked by the ECU via software algorithms, such as those employing speckle or feature tracking between consecutive images, for example, so as to ensure that the target tissue remains in the ROI.

Once the ROI has been confirmed, the method can continue at step 94 of delivering ablative HIFU energy to the target tissue in the ROI. The operator and/or ECU 16 can cause generator 20 to switch from an imaging mode (delivery of ultrasound energy) to a therapy mode (delivery of HIFU energy), and can control the duration of ablation therapy. Ablation can last for a period of milliseconds to seconds, for example. At step 95, a decision is made as to whether or not the ablation cycle is over. If it is not over, steps 90-94 can be repeated as many times as necessary to achieve accurate and appropriate treatment.

Once ablation ceases, the assessment mode can begin at step 96 with emission of electromagnetic radiation from the distal end of an optic fiber 30 toward tissue 12 to cause generation of one or more photoacoustic waves from tissue 12. ECU 16 may direct generator 20 to cease ablation of tissue 12 while concurrently directing electromagnetic radiation source 32 to generate electromagnetic radiation and deliver that radiation to tissue 12 through the optic fiber 30. Ablation creates a thermal lesion in tissue 12 with clearly recognizable optical and structural changes to tissue 12. In particular, the optical absorption of tissue 12 changes, and it is expected that this change is predictably similar across different patients. When electromagnetic radiation impinges on tissue 12, energy is absorbed by tissue 12 and adiabatically dissipated. The energy is converted to heat, leading to a transient thermoelastic expansion of tissue 12 which generates photoacoustic waves that can be detected by transducer 38. The contrast in optical absorption between healthy tissue and tissue that has been ablated produces differences in the waves that can be detected by transducer 38.For example, electromagnetic waves may only excite scar (ablated) tissue and not healthy tissue. Characteristics of the photoacoustic wave (e.g., magnitude, frequency, etc.) can be used to determine various characteristics of tissue 12, including lesion depth, size, and type. Ablation also creates a change in the coloration of tissue 12. This is the result of coagulation necrosis, shutting down microperfusion of the tissue 12. Healthy tissue may be brown-red, while scar or coagulated tissue is yellow-grey. The discoloration caused by ablation can be detected by photoacoustic imaging and used as an indicator of lesion depth.

The method can continue with the step 98 of generating a signal (e.g., a color of varying intensities shown on display 18 along with the 2D ultrasound images) indicative of a characteristic of tissue 12 responsive to the electromagnetic radiation. The detected characteristic may comprise a depth of a lesion in tissue 12, a size of a lesion in tissue 12, a type of a lesion in tissue 12, a degree of scaring or coagulation in tissue 12, a degree of conductivity in tissue 12, or a functionality of a lesion in tissue 12. The characteristic may also comprise a distance of tissue 12 from transducer 38.

The method can continue at step 100 of superimposing the tissue response signal due to the photoacoustic wave on the previously acquired 2D ultrasound image. The superimposed image can be displayed on display 18. The superimposed image can be used by the operator and/or ECU 16 to determine the effects of the ablation therapy in terms of lesion size, depth, location, etc. Based on these determined effects, at step 102 the operator and/or ECU 16 can determine whether ablation is complete for the current ROI. If ablation is not complete, the method can revert to step 90 of acquiring a 2D image of the ROI. If ablation is complete, the operator and/or ECU can choose a new ROI at step 104 (in which case the process reverts to step 86) or end the procedure at step 106. It should be understood that the steps of the method described herein can be performed in a repeated, iterative, fashion until, for example, the operator determines that sufficient ablation has occurred. It should also be noted that in some embodiments, assessment of the ablation therapy can be carried out via other methods in addition to or in place of photoacoustics.

Although several embodiments of this invention have been described above with a certain degree of particularity, those skilled in the art could make numerous alterations to the disclosed embodiments without departing from the scope of this invention. All directional references (e.g., upper, lower, upward, downward, left, right, leftward, rightward, top, bottom, above, below, vertical, horizontal, clockwise and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not as limiting. Changes in detail or structure may be made without departing from the invention as defined in the appended claims.

Although the various embodiments of the devices have been described herein in connection with certain disclosed embodiments, many modifications and variations to those embodiments may be implemented. For example, particular features, structures, or characteristics described above may be combined in any suitable manner in one or more embodiments. Thus, the particular features, structures, or characteristics illustrated or described in connection with one embodiment may be combined, in whole or in part, with the features structures, or characteristics of one or more other embodiments without limitation unless illogical or non-functional. Also, where materials are disclosed for certain components, other materials may be used. The foregoing description and following claims are intended to cover all such modification and variations.

## Claims

1. A system for assessing effects of ablation therapy on tissue in a body, comprising:
a) an ultrasound transducer (38) configured to generate ultrasound images and to deliver ultrasound ablative energy to tissue;
b) an optic fiber (30) configured to emit electromagnetic radiation to the tissue; and
c) an electronic control unit (ECU) (16) including the following:
an imaging module (74) configured to operate the ultrasound transducer in a first mode to generate and acquire at least one ultrasound image from the tissue within a region of interest within the body;
a tracking module (76) configured to identify and track the region of interest within the at least one ultrasound image and confirm that the tissue remains within the region of interest;
a therapy module (78) configured to generate a signal to cause the ultrasound transducer to operate in a second mode to deliver ultrasound ablative energy to the region of interest;
a radiation module (80) configured to generate a signal to cause the optic fiber to emit electromagnetic radiation to cause generation of a photoacoustic wave from tissue in the region of interest;
a tissue sensing module (82) configured to receive a tissue response signal indicative of a characteristic of tissue responsive to the electromagnetic radiation; and
an analysis module (84) configured to analyze the tissue response signal to determine at least one characteristic of the tissue responsive to the ablation therapy.

2. The system of claim 1, wherein the tracking module is further configured to identify and track the region of interest using the at least one ultrasound image acquired by the ultrasound transducer;

3. The system of claim 1 or 2, wherein the tissue sensing module (82) is configured to use the tissue response signal to control the therapy module.

4. The system of any one of claims 1 to 3, wherein the analysis module (84) is configured to overlay the tissue response signal and the ultrasound image.

5. The system of any one of claims 1 to 4, wherein the ultrasound ablative energy comprises high frequency ultrasound (HIFU) ablative energy.

6. The system of any one of claims 1 to 5, wherein the therapy module (78) is configured to operate during a first time period; wherein the radiation module (80) is configured to operate during a second time period; and wherein the ultrasound transducer (38) is configured to deliver the ultrasound ablative energy during the first time period and to receive the tissue response signal during the second time period.

7. The system of any one of claims 1 to 6, wherein the tracking module (76) is configured to identify and track the region of interest based on the position sensing electrodes (110, 112, 114).

8. The system of any one of claims 1 to 6, wherein the tracking module (76) is configured to use image speckle or feature tracking between a plurality of consecutive ultrasound images.

9. The system of any one of claims 1 to 8, wherein, while operating in the second mode, the tracking module (76) is configured to signal the ultrasound transducer to stop delivery of ultrasound ablative energy when the region of interest cannot be confirmed within the at least one ultrasound image.

10. The system of claim 1, comprising:
a first catheter (28) comprising:
an elongate shaft (62) having a proximal section and a distal section; wherein the ultrasound transducer (38) is disposed at the distal section of the shaft (62), and configured to deliver ultrasound ablative energy and to transmit and receive ultrasound waves that can be used to generate ultrasound images;
the optic fiber (30) is disposed within the shaft (62) of the first catheter (28) and configured to emit electromagnetic radiation from the distal section of the shaft (62); and
the electronic control unit (ECU) is in communication with the first catheter (28).

11. The system of claim 10, wherein the tracking module (76) is configured to identify and track the region of interest using the at least one ultrasound image acquired with the ultrasound transducer (38).

12. The system of claim 10, wherein the tissue sensing module (82) is configured to use the tissue response signal to control the therapy module (78).

13. The system of claim 10, wherein the analysis module (84) is configured to overlay the tissue response signal and the ultrasound image.

14. The system of claim 10, wherein the ultrasound ablative energy comprises high intensity focused ultrasound (HIFU) ablative energy.

15. The system of claim 10, wherein the therapy module (78) is configured to operate during a first time period; wherein the radiation module (80) is configured to operate during a second time period; and wherein the ultrasound transducer is configured to deliver the ultrasound ablative energy during the first time period and to receive the tissue response signal during the second time period.

## Patentansprüche

1. System zur Beurteilung der Wirkungen einer Ablationstherapie für Gewebe in einem Körper, mit:
a) einem Ultraschalltransducer (38), der konfiguriert ist zum Erzeugen von Ultraschallbildern und zum Liefern von Ultraschallablationsenergie an das Gewebe;
b) einer optischen Faser (30), die konfiguriert ist zum Aussenden elektromagnetischer Strahlung an das Gewebe; und
c) einer elektronischen Steuerungseinheit (ECU) (16), die aufweist:
ein Bildgebungsmodul (74), das konfiguriert ist zum Betreiben des Ultraschalltransducers in einem ersten Modus zum Erzeugen und Erfassen von mindestens einem Ultraschallbild von dem Gewebe innerhalb einer Region von Interesse innerhalb des Körpers;
ein Verfolgungsmodul (76), das konfiguriert ist zum Identifizieren und Verfolgen der Region von Interesse innerhalb des mindestens einen Ultraschallbilds und zum Bestätigen, dass das Gewebe in der Region von Interesse verbleibt;
ein Therapiemodul (78), das konfiguriert ist zum Erzeugen eines Signals, um den Ultraschalltransducer zu veranlassen in einem zweiten Modus zu arbeiten zur Lieferung von Ultraschallablationsenergie an die Region von Interesse;
ein Strahlungsmodul (80), das konfiguriert ist zum Erzeugen eines Signals, um die optische Faser zu veranlassen elektromagnetische Strahlung auszusenden zur Veranlassung der Erzeugung einer photoakustischen Welle von dem Gewebe in der Region von Interesse;
ein Gewebeerkennungsmodul (82), das konfiguriert ist zum Empfangen eines Gewebeantwortsignals, das kennzeichnend ist für eine Charakteristik des Gewebes in Antwort auf die elektromagnetische Strahlung; und
ein Analysemodul (84), das konfiguriert ist zur Analyse des Gewebeantwortsignals zur Bestimmung mindestens einer Eigenschaft des Gewebes in Reaktion auf die Ablationstherapie.

2. System nach Anspruch 1, bei dem das Verfolgungsmodul ferner konfiguriert ist zum Identifizieren und Verfolgen der Region von Interesse unter Verwendung des mindestens einen Ultraschallbilds, das von dem Ultraschalltransducer erfasst worden ist.

3. System nach Anspruch 1 oder 2, bei dem das Gewebeerkennungsmodul (82) konfiguriert ist zum Verwenden des Gewebeantwortsignals zur Steuerung des Therapiemoduls.

4. System nach einem der Ansprüche 1 bis 3, bei dem das Analysemodul (84) konfiguriert ist zum Überlagern des Gewebeantwortsignals und des Ultraschallbilds.

5. System nach einem der Ansprüche 1 bis 4, bei dem die Ultrachallablationsenergie eine Hochfrequenzultraschallablationsenergie (HIFU-Ablationsenergie) aufweist.

6. System nach einem der Ansprüche 1 bis 5, bei dem das Therapiemodul (78) konfiguriert ist, um während einer ersten Zeitperiode zu arbeiten; das Strahlungsmodul (80) konfiguriert ist, um während einer zweiten Zeitperiode zu arbeiten; und der Ultraschalltransducer (38) konfiguriert ist zum Liefern der Ultraschallablationsenergie während der ersten Zeitperiode und zum Empfangen des Gewebeantwortsignals während der zweiten Zeitperiode.

7. System nach einem der Ansprüche 1 bis 6, bei dem das Verfolgungsmodul (76) konfiguriert ist zum Identifizieren und Verfolgen der Region von Interesse basierend auf Positionserfassungselektroden (110, 112, 114).

8. System nach einem der Ansprüche 1 bis 6, bei dem das Erfassungsmodul (76) konfiguriert ist zur Verwendung eines Bildflecks oder einer Merkmalsverfolgung zwischen einer Mehrzahl von aufeinander folgenden Ultraschallbildern.

9. System nach einem der Ansprüche 1 bis 8, bei dem während eines Betriebs in dem zweiten Modus das Verfolgungsmodul (76) konfiguriert ist zum Signalisieren des Ultraschalltransducers die Lieferung der Ultraschallablationsenergie zu stoppen, wenn die Region von Interesse nicht in dem mindestens einen Ultraschallbild bestätigt ist.

10. System nach Anspruch 1, mit:
einem ersten Katheter (28), der aufweist:
einen länglichen Schaft (62) mit einem proximalen Abschnitt und einem distalen Abschnitt, wobei der Ultraschalltransducer (38) sich an dem distalen Abschnitt des Schafts (62) befindet und konfiguriert ist zur Lieferung der Ultraschallablationsenergie und zum Senden und Empfangen von Ultraschallwellen, die verwendet werden können zur Erzeugung der Ultraschallbilder; wobei die optische Faser (30) sich in dem Schaft (62) des ersten Katheters (28) befindet und konfiguriert ist zum Aussenden elektromagnetischer Strahlung von dem distalen Abschnitt des Schafts (62); und
die elektronische Steuerungseinheit (ECU) in Verbindung steht mit dem ersten Katheter (28).

11. System nach Anspruch 10, bei dem das Verfolgungsmodul (76) konfiguriert ist zum Identifizieren und Verfolgen der Region von Interesse unter Verwendung des mindestens einen Ultraschallbilds, das von dem Ultraschalltransducer (38) erfasst worden ist.

12. System nach Anspruch 10, bei dem das Gewebeerkennungsmodul (82) konfiguriert ist zur Verwendung des Gewebeantwortsignals zur Steuerung des Therapiemoduls (78).

13. System nach Anspruch 10, bei dem das Analysemodul (84) konfiguriert ist zum Überlagern des Gewebeantwortsignals und des Ultraschallbilds.

14. System nach Anspruch 10, bei dem die Ultraschallablationsenergie hochintensive, fokussierte Ultraschallablationsenergie (HIFU-Ablationsenergie) aufweist.

15. System nach Anspruch 10, bei dem das Therapiemodul (78) konfiguriert ist für einen Betrieb während einer ersten Zeitperiode; das Strahlungsmodul (80) konfiguriert ist für einen Betrieb während einer zweiten Zeitperiode; und der Ultraschalltransducer konfiguriert ist zur Lieferung der Ultraschallablationsenergie während der ersten Zeitperiode und zum Empfangen des Gewebeantwortsignals während der zweiten Zeitperiode.

## Revendications

1. Système pour évaluer les effets d'une thérapie d'ablation sur un tissu dans un corps, comprenant :
a) un transducteur à ultrasons (38) configuré pour générer des images ultrasonores et pour distribuer une énergie d'ablation à ultrasons à un tissu ;
b) une fibre optique (30) configurée pour émettre un rayonnement électromagnétique jusqu'au tissu ; et
c) une unité de commande électronique (ECU) (16) comportant ce qui suit :
un module d'imagerie (74) configuré pour faire fonctionner le transducteur à ultrasons dans un premier mode pour générer et acquérir au moins une image ultrasonore à partir du tissu à l'intérieur d'une région d'intérêt à l'intérieur du corps ;
un module de suivi (76) configuré pour identifier et suivre la région d'intérêt à l'intérieur de l'au moins une image ultrasonore et confirmer que le tissu reste dans la région d'intérêt ;
un module de thérapie (78) configuré pour générer un signal pour amener le transducteur à ultrasons à fonctionner dans un second mode pour distribuer une énergie d'ablation à ultrasons à la région d'intérêt ;
un module de rayonnement (80) configuré pour générer un signal pour amener la fibre optique à émettre un rayonnement électromagnétique pour provoquer la génération d'une onde photo-acoustique provenant d'un tissu dans la région d'intérêt ;
un module de détection de tissu (82) configuré pour recevoir un signal de réponse tissulaire indiquant une caractéristique d'un tissu en réponse au rayonnement électromagnétique ; et
un module d'analyse (84) configuré pour analyser le signal de réponse tissulaire pour déterminer au moins une caractéristique du tissu en réponse à la thérapie d'ablation.

2. Système selon la revendication 1, dans lequel le module de suivi est en outre configuré pour identifier et suivre la région d'intérêt en utilisant l'au moins une image ultrasonore acquise par le transducteur à ultrasons.

3. Système selon la revendication 1 ou 2, dans lequel le module de détection de tissu (82) est configuré pour utiliser le signal de réponse tissulaire pour commander le module de thérapie.

4. Système selon l'une quelconque des revendications 1 à 3, dans lequel le module d'analyse (84) est configuré pour superposer le signal de réponse tissulaire et l'image ultrasonore.

5. Système selon l'une quelconque des revendications 1 à 4, dans lequel l'énergie d'ablation à ultrasons comprend une énergie d'ablation à ultrasons haute fréquence (HIFU).

6. Système selon l'une quelconque des revendications 1 à 5, dans lequel le module de thérapie (78) est configuré pour fonctionner pendant un premier laps de temps ; dans lequel le module de rayonnement (80) est configuré pour fonctionner pendant un second laps de temps ; et dans lequel le transducteur à ultrasons (38) est configuré pour distribuer l'énergie d'ablation à ultrasons pendant le premier laps de temps et pour recevoir le signal de réponse tissulaire pendant le second laps de temps.

7. Système selon l'une quelconque des revendications 1 à 6, dans lequel le module de suivi (76) est configuré pour identifier et suivre la région d'intérêt sur la base d'électrodes de détection de position (110, 112, 114).

8. Système selon l'une quelconque des revendications 1 à 6, dans lequel le module de suivi (76) est configuré pour utiliser un suivi de tacheture ou de caractéristique d'image entre une pluralité d'images ultrasonores consécutives.

9. Système selon l'une quelconque des revendications 1 à 8, dans lequel, tout en fonctionnant dans le second mode, le module de suivi (76) est configuré pour signaler au transducteur à ultrasons d'arrêter la distribution d'énergie d'ablation à ultrasons lorsque la région d'intérêt ne peut être confirmée à l'intérieur de l'au moins une image ultrasonore.

10. Système selon la revendication 1, comprenant :
un premier cathéter (28) comprenant :
une tige allongée (62) présentant une section proximale et une section distale ; dans lequel le transducteur à ultrasons (38) est disposé sur la section distale de la tige (62), et configuré pour distribuer une énergie d'ablation à ultrasons et pour transmettre et recevoir des ondes ultrasonores qui peuvent être utilisées pour générer des images ultrasonores ;
la fibre optique (30) est disposée à l'intérieur de la tige (62) du premier cathéter (28) et configurée pour émettre un rayonnement électromagnétique à partir de la section distale de la tige (62) ; et
l'unité de commande électronique (ECU) est en communication avec le premier cathéter (28).

11. Système selon la revendication 10, dans lequel le module de suivi (76) est configuré pour identifier et suivre la région d'intérêt en utilisant l'au moins une image ultrasonore acquise avec le transducteur à ultrasons (38).

12. Système selon la revendication 10, dans lequel le module de détection de tissu (82) est configuré pour utiliser le signal de réponse tissulaire pour commander le module de thérapie (78).

13. Système selon la revendication 10, dans lequel le module d'analyse (84) est configuré pour superposer le signal de réponse tissulaire et l'image ultrasonore.

14. Système selon la revendication 10, dans lequel l'énergie d'ablation à ultrasons comprend une énergie d'ablation à ultrasons focalisés haute intensité (HIFU).

15. Système selon la revendication 10, dans lequel le module de thérapie (78) est configuré pour fonctionner pendant un premier laps de temps ; dans lequel le module de rayonnement (80) est configuré pour fonctionner pendant un second laps de temps ; et dans lequel le transducteur à ultrasons est configuré pour distribuer l'énergie d'ablation à ultrasons pendant le premier laps de temps et pour recevoir le signal de réponse tissulaire pendant le second laps de temps.
